# FASCICULE DE BREVET EUROPEEN

(11) **EP 1 092 420 B1**
(45) Date de publication et mention de la délivrance du brevet: **19.05.2004**
(21) Numéro de dépôt: 00402664.7
(22) Date de dépôt: 26.09.2000
(51) Int. Cl.: A61K 7/06

(54) **Composition de lavage des matières kératiniques, à base d'un agent tensio-actif détergent, d'un polymère cationique de vinyllactame et d'un terpolymère acrylique**
Haarshampoo auf Basis eines Tensids, ein kationisches Vinyllactampolymer und ein Acryl-Terpolymer
A washing composition for keratinous materials based on a surfactant, a cationic vinyllactam polymer and an acrylic terpolymer

(30) Priorité: 29.09.1999 FR 9912171
(43) Date de publication de la demande: 18.04.2001
(73) Titulaire: L'OREAL, 75008 Paris (FR)
(72) Inventeur: Maurin, Véronique, 75016 Paris (FR); Beauquey, Bernard, 92110 Clichy (FR)
(74) Mandataire: Casalonga, Axel

(56) Documents cités:
- WO-A-94/06403
- WO-A-95/01152
- WO-A-97/35545
- US-A- 5 910 472

## Description

La présente invention concerne d'une manière générale des compositions de lavage des matières kératiniques, à base d'un agent tensio-actif détergent, d'un polymère cationique de vinyllactame et d'un terpolymère acrylique, ainsi qu'un procédé de lavage mettant en oeuvre ces compositions.

Les polymères cationiques de vinyllactame et notamment les copolymères cationiques de vinyllactame comportant des motifs vinylimidazolium tels que les polymères vendus sous la dénomination générique "LUVIQUAT" par la société B.A.S.F., sont souvent utilisés dans des compositions de shampooings pour les qualités cosmétiques qu'ils apportent aux cheveux (voir par exemple WO-A-9 406 403, WO-A-9 501 152, WO-A-9 733 545, US-A-5 910 472).

Cependant, il a été constaté que ces copolymères cationiques présentaient souvent l'inconvénient de conduire à des performances cosmétiques insuffisantes sur des cheveux sensibilisés, principalement des cheveux sensibilisés mouillés, notamment en terme de douceur.

Il existe donc un besoin d'une composition cosmétique détergente, en particulier un shampooing, qui permette d'obtenir des performances cosmétiques acceptables sur les matières kératiniques, à savoir notamment les cheveux et le cuir chevelu, et plus particulièrement sur les cheveux sensibilisés.

La demanderesse a découvert, de façon surprenante, qu'il était possible de formuler des compositions de lavage des matières kératiniques, notamment des shampooings, améliorant plus particulièrement les propriétés cosmétiques des cheveux sensibilisés, en utilisant dans ces compositions un agent tensio-actif détergent et un copolymère cationique de vinyllactame associés à un terpolymère acrylique spécifique, défini ci-après. En effet, il a été constaté que l'utilisation dudit terpolymère acrylique dans les compositions de la présente invention permettait d'améliorer les propriétés cosmétiques des matières kératiniques et notamment des cheveux sensibilisés, particulièrement en apportant plus de douceur et de légèreté aux cheveux sensibilisés mouillés et plus de lissage, brillance et malléabilité aux cheveux sensibilisés séchés.

L'invention a donc pour objet des compositions de lavage des matières kératiniques essentiellement caractérisées en ce qu'elles comprennent dans un milieu cosmétiquement acceptable:
i) au moins un agent tensio-actif détergent;
ii) au moins un copolymère cationique de vinyllactame comprenant en outre au moins un motif de sel de méthylvinylimidazolium, de polyuréthane, de méthacrylamidopropyldiméthylamine, de méthacrylamidopropyltriméthylammonium ou de vinylcaprolactame; et
iii) au moins un terpolymère acrylique constitué:
   - de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
   - de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
   - de 0,1 à 30% en poids, préférentiellement de 0,1 à 10% en poids d'un monomère (c) choisi parmi :
      un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
      un monomère tensioactif insaturé éthylénique copolymérisable obtenu par condensation d'un tensioactif non-ionique avec un acide carboxylique insaturé α,β-éthylénique ou son anhydride;
      un monomère tensioactif choisi parmi les produits de réaction de type urée d'un monoisocyanate insaturé monoéthylénique avec un tensioactif non-ionique présentant une fonction amine;
      un éther de (méth)allyle de formule CH₂= CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur ou égal à 100, m et p désignent zéro ou un nombre entier inférieur à n et R₂ est un groupe hydrophobe d'au moins 8 atomes de carbone et préférentiellement de 8 à 30 atomes de carbone; et
      un monomère non-ionique de type uréthane produit par réaction d'un tensioactif non ionique monohydrique avec un isocyanate insaturé monoéthylénique;
      les pourcentages en poids de monomères étant basés sur le poids total des monomères constituant le terpolymère.

Dans la composition de lavage selon l'invention, le terpolymère acrylique est présent à raison de 0,01 à 20% en poids de matière active (M.A.), de préférence 0,1 à 10 % en poids, par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropylacrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou di-carboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes. Des monomères de réticulation peuvent notamment être des divinyl-benzènes des trivinyl-benzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octaallyl saccharoses, des cycloparrafines, des cyclooléfines et du N-méthylène-bis-acrylamide.

Les compositions conformes à l'invention comprennent en outre nécessairement un copolymère cationique de vinyllactame comprenant en outre au moins un motif de sel de méthylvinylimidazolium, de polyuréthane, de méthacrylamidopropyldi(tri)méthylamine(ammonium) , de méthacrylamidopropyltriméthylammonium ou de vinylcaprolactame.

Parmi les polymères comportant des motifs de vinylimidazolium on peut notamment citer:
- les polymères vinylpyrrolidone / chlorure de méthylvinylimidazolium, vendus sous les dénominations LUVIQUAT FC 370, FC 550, FC 905, HM 552, LUVIQUAT CARE, LUVIQUAT HOLD par la société B.A.S.F.;
- le polymère vinylpyrrolidone / chlorure de méthylvinylimidazolium / vinylimidazole, vendu sous la dénomination LUVIQUAT 8155 par la société B.A.S.F.; et
- les polymères vinylpyrrolidone / méthosulfate de méthylvinylimidazolium, vendu sous la dénomination LUVIQUAT MS 370 par la société B.A.S.F..

Les copolymères comportant des motifs polyuréthane sont notamment choisis parmi les polymères vinylpyrrolidone / méthacrylate de diméthylaminoéthyle / polyuréthane hydrophile, vendus sous la dénomination commerciale PECOGEL GC-310 par la société U.C.I.B. ou encore sous les dénominations AQUAMERE C 1031, C1011 et C1511 par la société BLAGDEN CHEMICALS.

Les polymères de vinylpyrrolidone comportant des motifs méthacrylamidopropyldiméthylamine peuvent notamment être choisis parmi les ACP1163, ACP1187, ACP1189, ACP1212, ACP1208 et ACP1213 de la société ISP.

Parmi les polymères de vinylpyrrolidone comportant des motifs méthacrylamidopropyltriméthylammonium (M.A.P.T.A.C.), on peut citer notamment les copolymères vinylpyrrolidone / M.A.P.T.A.C., vendus sous les dénominations commerciales GAFQUAT ACP 1011 et GAFQUAT HS 100 par la société I.S.P. et les terpolymères vinylpyrrolidone / M.A.P.T.A.C. / vinylcaprolactame, vendus sous les dénominations POLYMER ACP 1059, 1060 et 1156 par la société I.S.P..

Parmi les polymères comportant des motifs vinylcaprolactame, on peut citer notamment les terpolymères vinylpyrrolidone / méthacrylate de diméthylaminoéthyle / vinylcaprolactame, vendu sous les dénominations GAFFIXVC713, H2OLDEP1 et ADVANTAGE HC37 par la société I.S.P..

Dans le cadre de la présente invention, on préfère utiliser des copolymères cationiques de vinyllactame comprenant un motif vinylimidazolium.

Les polymères cationiques de vinyllactame peuvent être présents dans des concentrations en matière active comprises entre 0,005 et 10% en poids, de préférence entre 0,02% et 8% en poids et encore plus préférentiellement dans des proportions comprises entre 0,05% et 5% en poids, par rapport au poids total de la composition.

Comme indiqué précédemment, les compositions selon l'invention contiennent au moins un agent tensio-actif détergent, notamment choisi parmi les tensio-actifs anioniques, amphotères, non-ioniques et cationiques ayant des propriétés détergentes, et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates. Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éther carboxyliques polyoxyalkylénés ou leurs sels, les acides alkylamido éther carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par exemple chlorures) de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utilisé les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Les agents tensio-actifs sont utilisés dans les compositions conformes à l'invention dans des proportions suffisantes pour conférer un caractère détergent à la composition, généralement à raison d'au moins 4% en poids, de préférence entre 5 et 50% en poids par rapport au poids total de la composition et en particulier entre 8 et 35%.

Les compositions selon l'invention présentent un pH généralement compris entre 3 et 12, et plus particulièrement entre 4 et 8.

Le milieu cosmétiquement acceptable des compositions est constitué, soit par de l'eau, soit par un ou plusieurs solvants, soit par un mélange d'eau et d'au moins un solvant cosmétiquement acceptable choisi parmi les alcools inférieurs, les alkylèneglycols et les éthers de polyols.

Dans un mode de réalisation préféré de l'invention, les compositions selon la présente invention contiennent des polyorganosiloxanes modifiés ou non, à savoir des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDEou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges. On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHONE POULENC, l'huile "47 V 500 000" de RHONE POULENC ou certaines "VISCASIL" de GENERAL ELECTRIC, ou "MIRASIL" de RHONE POULENC et les polydiméthylsiloxanes linéaires à groupements terminaux hydroxydiméthylsilyle, telles que les huiles de la série 48 V de RHONE POULENC.
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 763" de RHONE POULENC;
   (iv) les gommes de silicone ; ce sont des polydiorganosiloxanes de masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges;elles ont, par exemple, les structures suivantes :
      - polydiméthylsiloxane,
      - poly[(diméthylsiloxane)/(méthylvinylsiloxane)],
      - poly[(diméthylsiloxane)/(diphénylsiloxane)],
      - poly[(diméthylsiloxane))(phénylméthylsiloxane)],
      - poly[(diméthylsiloxane)/(diphénylsiloxane)/(méthylvinylsiloxane)];
      on peut aussi citer, par exemple, à titre non limitatif, les mélanges suivants :
      1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
      2) les mélanges formés à partir d'une gomme polydiméthylsiloxane avec une silicone cyclique, tel que le produit "SF 1214 SILICONE FLUID" de GENERAL ELECTRIC, qui estune gomme SE 30 de PM 500 000 solubilisée dans la "SF 1202 SILICONE FLUID" (décaméthylcyclopentasiloxane);
      3) les mélanges de deux PDMS de viscosité différente, notamment d'une gomme PDMS et d'une huile PDMS, tels que les produits "SF 1236" et "CF 1241" de la Société GENERAL ELECTRIC;
   (v) les résines de silicone ; de préférence des systèmes siloxaniques réticulés renfermant les unités R₂SiO_{2/2}, RSiO_{3/2} et SiO_{4/2} dans lesquelles R représente un groupement hydrocarboné possédant 1 à 6 atomes de carbone ou un groupement phényle. Parmi ces résines, on peut citer le produit vendu sous la dénomination "DOW CORNING 593";
   (vi) les polyorganosiloxanes organomodifiés ; c'est-à-dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997);
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3270" de la Société GOLDSCHMIDT ;
      k) des groupements amphotères ou bétaïniques, tels que dans le produit vendu par la Société GOLDSCHMIDT sous la dénomination "ABIL B 9950" ;
      1) des groupements bisulfite, tels que dans les produits vendus par la Société GOLDSCHMIDT sous les dénominations "ABIL S 201" et "ABIL S 255";
   (vii) les copolymères blocs ayant un bloc linéaire polysiloxanepolyalkylène comme unité répétitive ; la préparation de tels copolymères blocs mis en oeuvre dans le cadre de la présente invention est décrite dans la demande européenne EP 0 492 657 A1, dont l'enseignement est inclus à titre de référence dans la présente description;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578 dont les enseignement sont totalement inclus dans la présente description à titre de références non limitatives;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776 dont les enseignements sont inclus totalement dans la présente description à titre de références non limitatives;
   (x) ou leurs mélanges.

Les polyorganosiloxanes préférés pour être utilisés selon l'invention sont les polyorganopolysiloxanes non volatils et préférentiellement les huiles ou gommes, les polydiméthylsiloxanes, les polydiméthylsiloxanes aminés, arylés ou alkylarylés.

Les polyorganosiloxanes sont utilisés dans les compositions de l'invention dans des proportions comprises entre 0,01 et 20% en poids et de préférence entre 0,1 et 10% en poids par rapport au poids total de la composition.

Selon un autre mode de réalisation préféré, les compositions de l'invention contiennent en outre au moins un polymère cationique différent des polymères cationiques de vinyllactame selon l'invention choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ.

Parmi les polymères cationiques susceptibles d'être utilisés dans le cadre de la présente invention, on préfère les dérivés d'éthers de cellulose comportant des groupements ammonium quaternaires et les cyclopolymères de méthyl diallyl amine ou de diméthyl diallyl ammonium.

Ces polymères cationiques sont utilisés dans les compositions de l'invention dans des proportions totales comprises entre 0,001 et 20% en poids et de préférence entre 0,5 et 5% en poids par rapport au poids total de la composition.

Les compositions selon l'invention peuvent également contenir en outre au moins un adjuvant choisi parmi les adjuvants habituellement utilisés en cosmétique, tels que les parfums, les conservateurs, les séquestrants, les humectants, les sucres, les huiles végétales, minérales, animales ou de synthèse, les polymères amphotères, le menthol, les dérivés de nicotinate, les agents anti-chute des cheveux, les agents anti-pelliculaires, les stabilisateurs de mousse, les agents propulseurs, les colorants, les filtres, les céramides, les vitamines ou provitamines, les agents acidifiants ou alcalinisants ou d'autres adjuvants cosmétiques bien connus.

Dans une forme de réalisation préférée de l'invention, les compositions selon l'invention sont utilisées comme shampooings pour le lavage des cheveux.

Le procédé de lavage des matières kératiniques consiste à appliquer une composition telle que définie ci-dessus sur des matières kératiniques humides ou sèches dans des quantités efficaces pour les laver, cette application étant suivie d'un rinçage après un temps de pose facultatif.

L' exemple qui suit est destiné à illustrer l'invention sans pour autant présenter un caractère limitatif.

### EXEMPLE I : SHAMPOOING

| | |
|---|---|
| Lauryl éther sulfate de sodium (2,2 OE) à 70% M.A. | 16 g |
| Cocoyl bétaïne en solution aqueuse à 30% | 6 g |
| Distéarate de glycol Copolymère de vinylpyrrolidone/ chlorure de méthyl vinyl imidazolium (5/95) en solution aqueuse à 40% de M.A. vendu sous la dénomination "LUVIQUAT | 2 g |
| FC 905" par la Société BASF | 0,75 g |
| Monoisopropanolamide d'acides de coprah Terpolymère d'acrylates, d'amino(méth)acrylates et d'itaconate d'alkyle en C₁₀-C₃₀ polyoxyéthyléné à 20 moles d'oxyde d'éthylène en dispersion aqueuse à 20% M.A. vendu sous la dénomination | 2,5 g |
| "STRUCTURE® PLUS" par la Société National Starch | 1 g |
| Conservateurs | qs |
| Eau déminéralisée stérilisée | qsp 100 g |

On ajuste le pH à 5,5 par de l'acide citrique ou par de la soude.

Afin d'évaluer les propriétés cosmétiques de ce shampooing, le même shampooing a été reproduit mais dans lequel les 0,75 g de "LUVIQUAT FC 905" ont été remplacés par 1,5 g de copolymère de vinylpyrrolidone/méthacrylate de diméthylaminoéthyl quaternisé par du sulfate de diéthyle en solution aqueuse à 20% de M.A., vendu sous la dénomination "GAFQUAT 755" par la Société I.S.P..
Après comparaison il a été constaté que les cheveux mouillés sensibilisés lavés avec le shampooing selon l'invention étaient plus doux et plus légers et que les cheveux sensibilisés séchés étaient plus lisses au toucher et présentaient plus de brillance et de malléabilité.

## Revendications

1. Composition de lavage des matières kératiniques, comprenant, dans un milieu cosmétiquement acceptable, au moins un agent tensio-actif détergent et au moins un copolymère cationique de vinyllactame comprenant en outre au moins un motif de sel de méthylvinylimidazolium, de polyuréthane, de méthacrylamidopropyldiméthylamine, de méthacrylamidopropyltriméthylammonium ou de vinylcaprolactame, **caractérisée par le fait qu'**elle comprend en outre au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di (C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non ionique présentant une fonctionnalité amine;
un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200, m et p désignent zéro ou un nombre entier inférieur à n et R₂ est un groupe hydrophobe d'au moins 8 atomes de carbone; et un monomère non-ionique de type uréthane produit par réaction d'un tensioactif non ionique monohydrique avec un isocyanate insaturé monoéthylénique;
les pourcentages en poids de monomères étant basés sur le poids total des monomères constituant le terpolymère.

2. Composition selon la revendication 1, **caractérisée en ce que** le terpolymère est présent à raison de 0,01 à 20% en poids de matière active, de préférence 0,1 à 10 % en poids par rapport au poids total de la composition.

3. Composition selon la revendication 1 ou 2, **caractérisée en ce que** le monomère (a) est choisi parmi les acrylates d'alkyle en C₂-C₆, et est préférentiellement l'acrylate d'éthyle.

4. Composition selon l'une quelconque des revendications 1 à 3, **caractérisée en ce que** le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropylméthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, **caractérisée en ce que** le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, **caractérisée en ce que** le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, **caractérisée en ce que** le terpolymère acrylique contient en outre un monomère de réticulation.

8. Composition selon l'une quelconque des revendications 1 à 7, **caractérisée en ce que** le copolymère cationique de vinyllactame est choisi parmi les polymères comportant des motifs de sel de méthylvinylimidazolium, et préférentiellement parmi les polymères vinylpyrrolidone / chlorure de méthylvinylimidazolium, les polymères vinylpyrrolidone / chlorure de méthylvinylimidazolium / vinylimidazole; et les polymères vinylpyrrolidone / méthosulfate de méthylvinylimidazolium.

9. Composition selon l'une quelconque des revendications 1 à 8, **caractérisée par le fait que** le copolymère cationique de vinyllactame est présent à une concentration de matière active de 0,005 à 10% en poids, de préférence de 0,02 à 8% en poids, et plus préférentiellement de 0,05 à 5% en poids, par rapport au poids total de la composition.

10. Composition selon l'une quelconque des revendications 1 à 9, **caractérisée en ce que** l'agent tensio-actif détergent est choisi parmi les agents tensio-actifs anioniques, amphotères, non-ioniques, cationiques et leurs mélanges.

11. Composition selon la revendication 10, **caractérisée par le fait que** les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylaryl-sulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs sels, les acides alkyl ou alkylaryl éther carboxyliques polyoxyalkylénés et leurs sels, les acides alkylamido éther carboxyliques polyoxyalkylénés et leurs sels.

12. Composition selon la revendication 10, **caractérisée par le fait que** les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

13. Composition selon la revendication 10, **caractérisée par le fait que** les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

14. Composition selon la revendication 10, **caractérisée par le fait que** les agents tensio-actifs cationiques sont choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

15. Composition selon l'une quelconque des revendications 1 à 14, **caractérisée en ce que** l'agent tensio-actif détergent est présent à raison d'au moins 4% en poids, de préférence de 5 à 50% en poids, et encore plus préférentiellement de 8 à 35% en poids par rapport au poids total de la composition.

16. Composition selon l'une quelconque des revendications 1 à 15, **caractérisée en ce qu'**elle présente un pH compris entre 3 et 12, et plus particulièrement entre 4 et 8.

17. Composition selon l'une quelconque des revendications 1 à 16, **caractérisée par le fait que** le milieu cosmétiquement acceptable est constitué par de l'eau ou par un ou plusieurs solvants ou par un mélange d'eau et d'au moins un solvant cosmétiquement acceptable choisi parmi les alcools inférieurs, les alkylèneglycols et les éthers de polyol.

18. Composition selon l'une quelconque des revendications 1 à 17, **caractérisée par le fait qu'**elle contient en outre au moins une silicone choisie parmi les silicones volatiles et les silicones non-volatiles et notamment parmi :
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes organomodifiés ;
(vii) les copolymères blocs ayant un bloc linéaire polysiloxane-polyalkylène comme unité répétitive ;
(vüi) les polymères siliconés greffés, à squelette organique non siliconé;
(ix) les polymères siliconés greffés, à squelette polysiloxanique greffé par des monomères organiques non siliconés;
(x) et leurs mélanges.

19. Composition selon la revendication 18, **caractérisée par le fait qu'**elle contient un polyorganosiloxane dans des proportions comprises entre 0,01 et 20% en poids et de préférence entre 0,1 et 10% en poids par rapport au poids total de la composition.

20. Composition selon l'une quelconque des revendications 1 à 19, **caractérisée par le fait qu'**elle contient en outre un polymère cationique différent des copolymères de vinyllactame choisi parmi les dérivés d'éthers de cellulose comportant des groupements ammonium quaternaires et les cyclopolymères de méthyl diallyl amine ou de diméthyl diallyl ammonium.

21. Composition selon l'une quelconque des revendications 1 à 20, **caractérisée par le fait qu'**elle contient en outre au moins un adjuvant cosmétiquement acceptable choisi parmi les parfums, les conservateurs, les séquestrants, les humectants, les sucres, les huiles végétales, minérales, animales ou de synthèse, les polymères amphotères, le menthol, les dérivés de nicotinate, les agents anti-chute de cheveux, les agents anti-pelliculaires, les stabilisateurs de mousse, les agents propulseurs, les colorants, les filtres, les céramides, les vitamines ou provitamines et les agents acidifiants ou alcalinisants.

22. Utilisation comme shampooing de la composition telle que définie dans l'une quelconque des revendications 1 à 21.

23. Procédé de lavage des matières kératiniques, **caractérisé par le fait que** l'on applique sur les matières kératiniques humides ou sèches au moins une composition telle que définie dans l'une quelconque des revendications 1 à 21, et après un temps de pose facultatif, on les rince à l'eau.

## Patentansprüche

1. Zusammensetzung zur Reinigung von Keratinsubstanzen, die in einem kosmetisch akzeptablen Medium mindestens einen reinigenden grenzflächenaktiven Stoff und mindestens ein kationisches Vinyllactam-Copolymer enthält, das ferner mindestens eine Methylvinylimidazoliumsalz-, Polyurethan-, Methacrylamidopropyldimethylamin-, Methacrylamidopropyltrimethylammonium- oder Vinylcaprolactam-Einheit aufweist, **dadurch gekennzeichnet, dass** sie ferner mindestens ein Acrylterpolymer enthält, das besteht aus:
- 5 bis 80 Gew.-%, vorzugsweise 15 bis 70 Gew.-% und noch bevorzugter 40 bis 70 Gew.-% eines Acrylatmonomers (a), das unter den C₁₋₆-Alkylacrylaten und C₁₋₆-Alkylmethacrylaten ausgewählt ist;
- 5 bis 80 Gew.-%, vorzugsweise 10 bis 70 Gew.-% und noch bevorzugter 20 bis 60 Gew.-% eines Monomers (b), das unter den heterocyclischen Vinylverbindungen, die mindestens ein Stickstoffatom oder Schwefelatom enthalten, (Meth)acrylamiden, Mono- oder Di-C₁₋₄-alkylamino-C₁₋₄alkyl(meth)acrylaten und Mono- oder Di-C₁₋₄-Alkylamino-C₁₋₄-alkyl(meth)-acrylamiden ausgewählt ist;
- 0,1 bis 30 Gew.-% und vorzugsweise 0,1 bis 10 Gew.-% eines Monomers (c), das ausgewählt ist unter:
· Urethanen, die bei der Umsetzung eines monoethylenisch ungesättigten Isocyanats mit einem nichtionischen grenzflächenaktiven Stoff, der ein Sequenzcopolymer von 1,2-Butylenoxid und Ethylenoxid mit endständiger C₁₋₄-Alkoxygruppe einschließt, entsteht;
· copolymerisierbaren, ethylenisch ungesättigten Tensidmonomeren, die bei der Kondensation von nichtionischen grenzflächenaktiven Stoffen mit α,β-ethylenisch ungesättigten Carbonsäuren oder ihren Anhydriden gebildet werden;
Tensidmonomeren, die unter den Reaktionsprodukten vom Harnstofftyp von monoethylenisch ungesättigten Monoisocyanaten mit nichtionischen grenzflächenaktiven Stoffen, die eine Aminofunktion aufweisen, ausgewählt sind;
· (Meth)allylethern der Formel CH₂=CR₁CH₂OAₘBₙAₚR₂, wobei in der Formel R, ein Wasserstoffatom oder die Methylgruppe bedeutet, A eine Propylenoxy- oder Butylenoxygruppe bezeichnet, B Ethylenoxy ist, n Null oder eine ganze Zahl von höchstens 200 und m und p Null oder eine ganze Zahl unter n bedeuten und R₂ eine hydrophobe Gruppe mit mindestens 8 Kohlenstoffatomen ist; und
· nichtionischen Monomeren vom Urethantyp, die bei der Reaktion von nichtionischen grenzflächenaktiven Stoffen mit einer Hydroxygruppe und monoethylenisch ungesättigten Isocyanaten gebildet werden;
wobei die prozentualen Gewichtsanteile der Monomere auf dem Gesamtgewicht der Monomere, die das Terpolymer bilden, basieren.

2. Zusammensetzung nach Anspruch 1, **dadurch gekennzeichnet, dass** das Terpolymer in einer Menge von 0,01 bis 20 Gew.-% Wirkstoff und vorzugsweise 0,1 bis 10 Gew.-%, bezogen auf das Gesamtgewicht der Zusammensetzung, vorliegt.

3. Zusammensetzung nach Anspruch 1 oder 2, **dadurch gekennzeichnet, dass** das Monomer (a) unter den C₂₋₆-Alkylacrylaten ausgewählt ist und es sich vorzugsweise um Ethylacrylat handelt.

4. Zusammensetzung nach einem der Ansprüche 1 bis 3, **dadurch gekennzeichnet, dass** das Monomer (b) unter N,N-Dimethylaminoethylmethacrylat, N,N-Diethylaminoethylacrylat,
N,N-Diethylaminoethylmethacrylat, N-*t*-Butylaminoethylacrylat, N-*t*-Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid und vorzugsweise dem N,N-Dimethylaminoethylmethacrylat ausgewählt ist.

5. Zusammensetzung nach einem der Ansprüche 1 bis 4, **dadurch gekennzeichnet, dass** das Monomer (c) ein copolymerisierbares, ethylenisch ungesättigtes Tensidmonomer ist, das durch Kondensation eines nichtionischen grenzflächenaktiven Stoffes mit Itaconsäure entsteht.

6. Zusammensetzung nach einem der Ansprüche 1 bis 5, **dadurch gekennzeichnet, dass** das Acrylterpolymer aus Acrylaten, Amino(meth)acrylaten und mit 20 mol Ethylenoxid polyethoxyliertem C₁₀₋₃₀-Alkylitaconat besteht.

7. Zusammensetzung nach einem der Ansprüche 1 bis 6, **dadurch gekennzeichnet, dass** das Acrylterpolymer ferner ein Monomer zur Vernetzung enthält.

8. Zusammensetzung nach einem der Ansprüche 1 bis 7, **dadurch gekennzeichnet, dass** das kationische Vinyllactampolymer unter den Polymeren, die Methylvinylimidazoliumsalz-Einheiten enthalten, und insbesondere unter den Vinylpyrrolidon/Methylvinylimidazoliumchlorid-Polymeren, Vinylpyrrolidon/Methylvinylimidazoliumchlorid/Vinylimidazol-Polymeren und Vinylpyrrolidon /Methylvinylimidazoliummethosulfat-Polymeren ausgewählt ist.

9. Zusammensetzung nach einem der Ansprüche 1 bis 9, **dadurch gekennzeichnet, dass** kationische Vinyllactam-Copolymer in einer Wirkstoffkonzentration von 0,005 bis 10 Gew.-%, vorzugsweise 0,02 bis 8 Gew.-% und noch bevorzugter 0,05 bis 5 %, bezogen auf das Gesamtgewicht der Zusammensetzung, enthalten ist.

10. Zusammensetzung nach einem der Ansprüche 1 bis 10, **dadurch gekennzeichnet, dass** der reinigende grenzflächenaktive Stoff unter den anionischen, amphoteren, nichtionischen und kationischen grenzflächenaktiven Stoffen und deren Gemischen ausgewählt ist.

11. Zusammensetzung nach Anspruch 10, **dadurch gekennzeichnet, dass** die anionischen grenzflächenaktiven Stoffe unter den Alkalisalzen, Magnesiumsalzen, Ammoniumsalzen, Aminsalzen oder Aminoalkoholsalzen der folgenden Verbindungen: Alkylsulfate, Alkylethersulfate, Alkylamidoethersulfate, Alkylarylpolyethersulfate, Monoglyceridsulfate; Alkylsulfonate, Alkylamidsulfonate, Alkylarylsulfonate, Olefinsulfonate, Paraffinsulfonate; Alkylsulfosuccinate, Alkylethersulfosuccinate, Alkylamidsulfosuccinate; Alkylsulfosuccinamate; Alkylsulfoacetate; Alkylphosphate, Alkyletherphosphate; Acylsarcosinate, Acylisethionate und N-Acyltaurate; wobei die Alkyl- oder Acylgruppen dieser verschiedenen Verbindungen aus einer Kohlenstoffkette mit 8 bis 30 Kohlenstoffatomen bestehen; Salzen von Ölsäure, Ricinolsäure, Palmitinsäure und Stearinsäure; Säuren von Kopraöl oder hydriertem Kopraöl; Acyllactylaten, deren Acylgruppe 8 bis 30 Kohlenstoffatome aufweist; Alkyl-D-galactosiduronsäuren und ihren Salzen, polyalkoxylierten Carbonsäurealkylethem oder Carbonsäurealkylarylethern oder ihren Salzen und polyalkoxylierten Carbonsäurealkylamidoethern oder ihren Salzen ausgewählt sind.

12. Zusammensetzung nach Anspruch 10, **dadurch gekennzeichnet, dass** die nichtionischen grenzflächenaktiven Stoffe unter den Alkoholen oder Alkylphenolen oder Fettsäuren, die polyethoxyliert, polypropoxyliert oder mehrfach mit Glycerin verethert sind und eine Fettkette mit 8 bis 30 Kohlenstoffatomen aufweisen, wobei die Zahl der Ethylenoxid- und Propylenoxidgruppen im Bereich von 2 bis 50 und die Anzahl der Glyceringruppen im Bereich von 2 bis 30 liegt; den Copolymeren von Ethylenoxid und Propylenoxid; den Kondensaten von Ethylenoxid und Propylenoxid mit Fettalkoholen; polyethoxylierten Fettamiden; mehrfach mit Glycerin veretherten Fettamiden; polyethoxylierten Fettaminen; ethoxylierten Sorbitanfettsäureestern; Saccharosefettsäureestern oder Polyethylenglykolfettsäureestern; Alkylpolyglykosiden; Amid- oder Carbamatderivaten von N-Alkylglucaminen, Aldobionamiden und Aminoxiden ausgewählt sind.

13. Zusammensetzung nach Anspruch 10, **dadurch gekennzeichnet, dass** die amphoteren grenzflächenaktiven Stoffe unter den aliphatischen, sekundären oder tertiären Aminderivaten, deren aliphatische Gruppe eine lineare oder verzweigte Kette mit 8 bis 22 Kohlenstoffatomen ist, die mindestens eine wasserlösliche anionische Gruppe, Carboxylat, Sulfonat, Sulfat, Phosphat oder Phosphonat aufweist; C₈₋₂₀-Alkylbetainen; Sulfobetainen, C₈₋₂₀-Alkyl-C₁₋₆amidoalkylbetainen oder C₈₋₂₀-Alkyl-C₁₋₆-amidoalkylsulfobetainen ausgewählt sind.

14. Zusammensetzung nach Anspruch 10, **dadurch gekennzeichnet, dass** die kationischen grenzflächenaktiven Stoffe unter den primären, sekundären oder tertiären, gegebenenfalls polyalkoxylierten Aminsalzen; quartären Ammoniumsalzen; Imidazolinderivaten; und Aminoxiden mit kationischem Charakter ausgewählt sind.

15. Zusammensetzung nach einem der Ansprüche 1 bis 14, **dadurch gekennzeichnet, dass** der reinigende grenzflächenaktive Stoff in einer Menge von mindestens 4 Gew.-%, vorzugsweise 5 bis 50 Gew.-% und besonders bevorzugt 8 bis 35 Gew.-%, bezogen auf das Gesamtgewicht der Zusammensetzung, vorliegt.

16. Zusammensetzung nach einem der Ansprüche 1 bis 15, **dadurch gekennzeichnet, dass** sie einen pH-Wert im Bereich von 3 bis 12 und insbesondere 4 bis 8 aufweist.

17. Zusammensetzung nach einem der Ansprüche 1 bis 16, **dadurch gekennzeichnet, dass** das kosmetisch akzeptable Medium aus Wasser oder einem oder mehreren Lösungsmitteln oder einem Gemisch von Wasser und mindestens einem kosmetisch akzeptablen Lösungsmittel besteht, das unter den niederen Alkoholen, Alkylenglykolen und Polyolethern ausgewählt ist.

18. Zusammensetzung nach einem der Ansprüche 1 bis 17, **dadurch gekennzeichnet, dass** sie ferner mindestens ein Silicon enthält, das unter den flüchtigen Siliconen und nicht flüchtigen Siliconen und insbesondere den folgenden Verbindungen ausgewählt ist:
(i) Polyalkylsiloxanen;
(ii) Polyarylsiloxanen;
(iii) Polyalkylarylsiloxanen;
(iv) Silicongummis;
(v) Siliconharzen;
(vi) organomodifizierten Polyorganosiloxanen;
(vii) Blockcopolymeren, die als wiederkehrende Einheit einen linearen Polysiloxan-Polyalkylen-Block enthalten;
(viii) gepfropften Siliconpolymere mit organischem, nicht siliconhaltigen Grundgerüst;
(ix) gepfropften Siliconpolymere mit Polysiloxangrundgerüst, auf das nicht siliconhaltige, organische Monomere gepfropft sind;
(x) und deren Gemischen.

19. Zusammensetzung nach Anspruch 18, **dadurch gekennzeichnet, dass** sie ein Polyorganosiloxan in Mengenanteilen von 0,01 bis 20 Gew.-% und vorzugsweise 0,1 bis 10 Gew.-%, bezogen auf das Gesamtgewicht der Zusammensetzung, enthält.

20. Zusammensetzung nach einem der Ansprüche 1 bis 19, **dadurch gekennzeichnet, dass** sie ein kationisches Polymer enthält, das von den Vinyllactam-Copolymeren verschieden ist und das unter den Celluloseetherderivaten mit quartären Ammoniumgruppen und den Methyldiallylamin-Cyclopolymeren oder Dimethyldiallylammonium-Cyclopolymeren ausgewählt ist.

21. Zusammensetzung nach einem der Ansprüche 1 bis 20, **dadurch gekennzeichnet, dass** sie außerdem mindestens einen kosmetisch akzeptablen Zusatzstoff enthält, der ausgewählt ist unter: Parfums, Konservierungsmitteln, Maskierungsmitteln, Feuchthaltemitteln, Zuckern, pflanzlichen, mineralischen, tierischen oder synthetischen Ölen, amphoteren Polymeren, Menthol, Nicotinatderivaten, Wirkstoffen gegen Haarausfall, Wirkstoffen gegen Schuppen, Schaumstabilisatoren, Treibmitteln, Farbmitteln, Filtern, Ceramiden, Vitaminen oder Provitaminen und Ansäuerungs- oder Alkalisierungsmitteln.

22. Verwendung der Zusammensetzung nach einem der Ansprüche 1 bis 21 als Haarwaschmittel.

23. Verfahren zur Reinigung von Keratinsubstanzen, **dadurch gekennzeichnet, dass** auf die feuchten oder trockenen Keratinsubstanzen mindestens eine Zusammensetzung nach einem der Ansprüche 1 bis 21 aufgetragen und nach einer fakultativen Einwirkzeit mit Wasser gespült wird.

## Claims

1. Composition for washing keratin materials, comprising, in a cosmetically acceptable medium, at least one detergent surfactant and at least one cationic vinyllactam copolymer also comprising at least one methylvinylimidazolium salt, polyurethane, methacrylamidopropyldimethylamine, methacrylamidopropyltrimethylammonium or vinylcaprolactam unit, **characterized in that** it also comprises at least one acrylic terpolymer consisting of:
- from 5% to 80% by weight, preferably from 15% to 70% by weight and more preferably from 40% to 70% by weight, of an acrylate monomer (a) chosen from a C₁-C₆ alkyl acrylate and a C₁-C₆ alkyl methacrylate;
- from 5% to 80% by weight, preferably from 10% to 70% by weight and more preferably from 20% to 60% by weight, of a monomer (b) chosen from a heterocyclic vinyl compound containing at least one nitrogen or sulphur atom, a (meth)acrylamide, a mono- or di (C₁-C₄) alkylamino (C₁-C₄) alkyl (meth) acrylate and a mono- or di (C₁-C₄)alkylamino(C₁-C₄) alkyl (meth) acrylamide;
- from 0.1% to 30% by weight, preferably from 0.1% to 10% by weight, of a monomer (c) chosen from:
a urethane produced by reaction between a
monoethylenic unsaturated isocyanate and a nonionic surfactant encompassing a block copolymer of 1,2-butylene oxide and of ethylene oxide containing a C₁₋₄ alkoxy end;
a copolymerizable ethylenic unsaturated surfactant monomer obtained by condensing a nonionic surfactant with an α,β-ethylenic unsaturated carboxylic acid or its anhydride;
a surfactant monomer chosen from reaction products such as urea of a monoethylenic unsaturated monoisocyanate with a nonionic surfactant containing an amine functionality;
a (meth)allyl ether of formula CH₂=CR₁CH₂OAₘBₙAₚR₂ in which R₁ denotes a hydrogen atom or a methyl group, A denotes a propylenoxy or butylenoxy group, B denotes ethylenoxy, n is equal to zero or denotes an integer less than or equal to 200, m and p denote zero or an integer less than n and R₂ is a hydrophobic group of at least 8 carbon atoms and preferably of C₈-C₃₀; and
a nonionic monomer such as urethane produced by reaction of a monohydric nonionic surfactant with a monoethylenic unsaturated isocyanate;
the weight percentages of monomers being based on the total weight of the monomers constituting the terpolymer.

2. Composition according to Claim 1, **characterized in that** the terpolymer is present in a proportion of from 0.01% to 20% by weight of active material, preferably 0.1% to 10% by weight, relative to the total weight of the composition.

3. Composition according to Claim 1 or 2, **characterized in that** the monomer (a) is chosen from C₂-C₆ alkyl acrylates and is preferably ethyl acrylate.

4. Composition according to any one of Claims 1 to 3, **characterized in that** the monomer (b) is chosen from N,N-dimethylaminoethyl methacrylate, N,N-diethylaminoethyl acrylate, N,N-diethylaminoethyl methacrylate, N-t-butylaminoethyl acrylate, N-t-butylaminoethyl methacrylate, N,N-dimethylaminopropylacrylamide, N,N-dimethylaminopropylmethacrylamide, N,N-diethylaminopropylacrylamide and N,N-diethylaminopropylmethacrylamide and is preferably N,N-dimethylaminoethyl methacrylate.

5. Composition according to any one of Claims 1 to 4, **characterized in that** the monomer (c) is a copolymerizable ethylenic unsaturated surfactant monomer obtained by condensing a nonionic surfactant with itaconic acid.

6. Composition according to any one of Claims 1 to 5, **characterized in that** the acrylic terpolymer consists of acrylates, amino (meth) acrylates and C₁₀-C₃₀ alkyl itaconate, polyoxyethylenated with 20 mol of ethylene oxide.

7. Composition according to any one of Claims 1 to 6, **characterized in that** the acrylic terpolymer also contains a crosslinking monomer.

8. Composition according to any one of Claims 1 to 7, **characterized in that** the cationic vinyllactam copolymer is chosen from polymers comprising methylvinylimidazolium salt units, and preferably from vinylpyrrolidone/methylvinylimidazolium chloride polymers, vinylpyrrolidone/methylvinylimidazolium chloride/vinylimidazole polymers and vinylpyrrolidone/methylvinylimidazolium methosulphate polymers.

9. Composition according to any one of Claims 1 to 8, **characterized in that** the cationic vinyllactam copolymer is present in an active material concentration of from 0.005% to 10% by weight, preferably from 0.02% to 8% by weight and more preferably from 0.05% to 5% by weight, relative to the total weight of the composition.

10. Composition according to any one of Claims 1 to 9, **characterized in that** the detergent surfactant is chosen from anionic, amphoteric, nonionic and cationic surfactants, and mixtures thereof.

11. Composition according to Claim 10, **characterized in that** the anionic surfactants are chosen from alkaline salts, magnesium salts, ammonium salts, amine salts amino alcohol salts of the following compounds: alkyl sulphates, alkyl ether sulphates, alkylamido ether sulphates, alkylaryl polyether sulphates, monoglyceride sulphates; alkyl sulphonates, alkylamide sulphonates, alkylaryl sulphonates, olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl phosphates, alkyl ether phosphates; acyl sarcosinates, acyl isethionates and N-acyl taurates; the alkyl or acyl radical in these various compounds generally consists of a carbon-based chain containing from 8 to 30 carbon atoms; fatty acid salts of oleic, ricinoleic, palmitic and stearic acid; coconut oil acid or hydrogenated coconut oil acid; acyl lactylates, in which the acyl radical contains from 8 to 30 carbon atoms; alkyl D-galactosiduronic acids and their salts, polyoxyalkylenated alkyl or alkylaryl ether carboxylic acids or their salts, and polyoxyalkylenated alkylamido ether carboxylic acids and their salts.

12. Composition according to Claim 10, **characterized in that** the nonionic surfactants are chosen from polyethoxylated, polyoxypropylenated or polyglycerolated fatty acids or alkylphenols or alcohols, with a fatty chain containing 8 to 30 carbon atoms, the number of ethylene oxide or propylene oxide groups being between 2 and 50 and the number of glycerol groups being between 2 and 30; copolymers of ethylene oxide and propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose, fatty acid esters of polyethylene glycol; alkylpolyglycosides; carbamate or amide derivatives of N-alkylglucamines, aldobionamides and amine oxides.

13. Composition according to Claim 10, **characterized in that** the amphoteric surfactants are chosen from secondary or tertiary aliphatic amine derivatives, in which the aliphatic radical is a linear or branched chain containing 8 to 22 carbon atoms and which contains at least one carboxylate, sulphonate, sulphate, phosphate or phosphonate water-solubilizing anionic group; (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines.

14. Composition according to Claim 10, **characterized in that** the cationic surfactants are chosen from salts of optionally polyoxyalkylenated primary, secondary or tertiary fatty amines; quaternary ammonium salts; imidazoline derivatives; or amine oxides of cationic nature.

15. Composition according to any one of Claims 1 to 14, **characterized in that** the detergent surfactant is present in a proportion of at least 4% by weight, preferably from 5% to 50% by weight and even more preferably from 8% to 35% by weight, relative to the total weight of the composition.

16. Composition according to any one of Claims 1 to 15, **characterized in that** it has a pH of between 3 and 12 and even more particularly between 4 and 8.

17. Composition according to any one of Claims 1 to 16, **characterized in that** the cosmetically acceptable medium consists of water, of one or more solvents or of a mixture of water and at least one solvent chosen from lower alcohols, alkylene glycols and polyol ethers.

18. Composition according to any one of Claims 1 to 17, **characterized in that** it also contains at least one silicone chosen from volatile silicones and non-volatile silicones, and in particular from:
(i) polyalkylsiloxanes;
(ii) polyarylsiloxanes;
(iii) polyalkylarylsiloxanes;
(iv) silicone gums;
(v) silicone resins;
(vi) organomodified polyorganosiloxanes;
(vii) block copolymers containing a polysiloxane-polyalkylene block as repeating unit;
(viii) grafted silicone polymers, containing a non-silicone organic skeleton;
(ix) grafted silicone polymers, containing a polysiloxane skeleton grafted with non-silicone organic monomers;
(x) and mixtures thereof.

19. Composition according to Claim 18, **characterized in that** it contains a polyorganosiloxane in proportions of between 0.01% and 20% by weight and preferably between 0.1% and 10% by weight relative to the total weight of the composition.

20. Composition according to any one of Claims 1 to 19, **characterized in that** it also contains a cationic polymer other than vinyllactam copolymers, chosen from cellulose ether derivatives comprising quaternary ammonium groups and methyldiallylamine cyclopolymers or dimethyldiallylammonium cyclopolymers.

21. Composition according to any one of Claims 1 to 20, **characterized in that** it also contains at least one cosmetically acceptable adjuvant chosen from fragrances, preserving agents, sequestering agents, wetting agents, sugars, plant, animal, mineral or synthetic oils, amphoteric polymers, menthol, nicotinate derivatives, agents for preventing hair loss, antidandruff agents, foam stabilizers, propellants, dyes, screening agents, ceramides, vitamins or provitamins and acidifying or basifying agents.

22. Use, as a shampoo, of the composition as defined in any one of Claims 1 to 21.

23. Process for washing keratin materials, **characterized in that** at least one composition as defined in any one of Claims 1 to 21 is applied to the wet or dry keratin materials and, after an optional period of leaving to stand on these materials, they are rinsed with water.
